# EUROPEAN PATENT APPLICATION

(11) **EP 4 745 120 A1**
(43) Date of publication of application: **20.05.2026**
(21) Application number: 24213022.7
(22) Date of filing: 14.11.2024
(51) Int. Cl.: C07C 271/00, C08G 18/10, C08G 18/28, C08G 18/32, C08G 18/48, C08G 18/67, C08G 18/73, C08G 18/75, C08G 18/78, C08G 18/80, C08G 18/81, C08G 71/04

(54) **EXO-VINYLENE CARBONATE COMPOUNDS AS PRECURSORS FOR ISOCYANATE-FREE URETHANE-TYPE POLYMERS**

(71) Applicant: BASF SE, 67056 Ludwigshafen am Rhein (DE); Albert-Ludwigs-Universität Freiburg, 79098 Freiburg (DE)
(72) Inventor: RUDOLF, Peter, 67056 Ludwigshafen am Rhein (DE); FUKUZUMI, Takeo, 67056 Ludwigshafen am Rhein (DE); MERKENS, Kay, 41199 Moenchengladbach (DE); RUEHE, Juergen, 79110 Freiburg (DE); KONRADI, Rupert, 67056 Ludwigshafen am Rhein (DE)
(74) Representative: Meissner Bolte Partnerschaft mbB

(57) **Abstract**

A compound of formula (I) 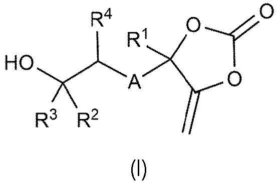 wherein R¹ is H or methyl, R² and R³ are independently selected from H, C₁-C₄ alkyl, cycloalkyl and phenyl-C₁-C₄-alkyl, or R² and R³, together with the carbon atom to which they are attached, form a C₃-C₆ carbocycle, R⁴ is H or C₁-C₄ alkyl, and A is C₂-C₇-alkylene is a suitable precursor for polyisocyanate-adducts and crosslinkers for obtaining isocyanate-free urethane-type polymers.

## Description

The present invention relates to exo-vinylene carbonate compounds as precursors for isocyanate-free urethane-type polymers.

Polyurethanes are versatile materials and have been used in a wide variety of applications such as foam insulation, car seats, adhesives, tubing and cabling elastomers, paint coatings and abrasion resistant coatings. Polyurethanes may be used in protective coatings (e.g. to wood, metal, plastic), adhesives to rigid substrates (e.g. composites, metal), adhesives to flexible substrates (textile, plastic film), in applications that require moisture-resistance (e.g. in outdoor use, in sealing, in electronics), and in tough and wear-resistant elastomers.

Conventionally, polyurethanes are manufactured by the reaction of polyols with isocyanates, preferably in the presence of a catalyst. Such isocyanates, in particular readily volatile and/or migrating monomeric diisocyanates, are considered toxic. Materials that are, or are suspected to be, ecologically damaging are increasingly unacceptable and alternative ecologically safer solutions are demanded. Furthermore, isocyanates are highly reactive and prone to an undesirable side-reaction between the isocyanate groups with or without moisture. Reaction with moisture yields carbon dioxide within the polyurethane mass, resulting in bubbles of carbon dioxide being trapped in the finished material, causing the polyurethane to be porous. Moreover, the curing reaction is typically catalyzed by a polyaddition catalyst such as a tin catalyst. Due to their high toxicity, the exposure of end-users to such tin catalysts is undesirable.

One alternative to isocyanate-based polyurethanes is provided by the essentially isocyanate-free polyurethanes, often abbreviated as NIPU (non isocyanate polyurethane). Such isocyanate-free polyurethanes are produced without the use of toxic isocyanates. A known method is, for example, the production of isocyanate-free polyurethanes from dicarbonates having cyclic carbonate groups and diamines by ring opening reaction addition. However, this reaction is associated with some drawbacks. The curing does not proceed at ambient conditions and the ring opening is non-selective. Neither primary amines, secondary amines nor alcohols as crosslinkers will inevitably result in the formation of urethane bonds. If urethane groups are formed, the reaction results in polymers that contain hydroxyl groups in the β-position to the urethane group and which have increased hydrophilicity.

Exo-vinylene carbonates can overcome these drawbacks by showing much higher reactivity and selectivity while avoiding the formation of hydroxyl groups.

US 2020/0255587 A1 describes a hydrocarbon-based copolymer comprising two exo-vinylene cyclocarbonate end groups. A mixture of the copolymer with an amine compound comprising at least two amine groups is said to be functional as an adhesive.

EP 3299402 discloses a two-component sealant and/or adhesive composition comprising i) in a first component, a compound having two or more exo-vinylene cyclic carbonate units, and, ii) in a second component, a multifunctional hardener that has at least two functional groups selected from primary amino groups, secondary amino groups, hydroxy groups, phosphine groups, phosphonate groups, and mercaptan groups.

The reaction of nucleophiles with exo-vinylene carbonates does not, or at least does not necessarily, result in the formation of urethane bonds. The reaction of secondary amines yields carbamates while primary amines can show more complex reaction behavior, mainly depending on the polarity of the matrix, temperature and the substitution pattern of the 5-membered ring. This means that neither primary amines, secondary amines nor alcohols as crosslinkers will inevitably result in the formation of urethane bonds. Hence, the resulting polymers lack hydrogen bond forming character which is a crucial element for the performance of "classic polyurethane chemistry".

DE1145632B discloses the preparation of 5-methylene-4,4-dialkyl-1,3-dioxolan-2-ones by reaction of acetylene alcohols with carbon dioxide in the presence of copper salts as catalysts.

WO2016202652A1 discloses the reaction sequence shown below comprising reacting hydroxypentanone with acetylene to obtain a diol, reacting of the diol with formic acid to obtain a formate, and reacting the formate with carbon dioxide to obtain 3-(4-methyl-5-methylene-2-oxo-1,3-dioxolan-4-yl)propyl formate.

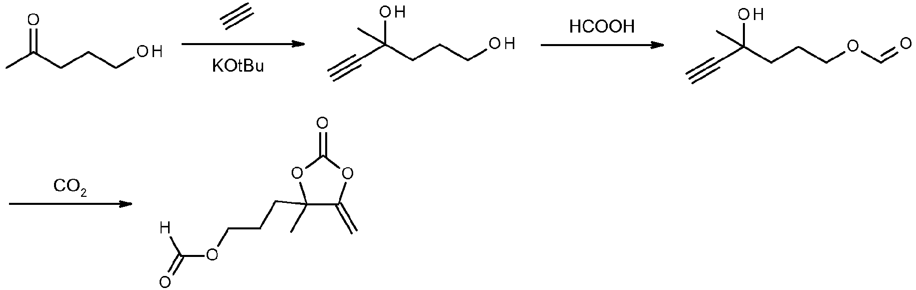

Crosslinking reactions require exo-vinylene carbonate compounds with more than one exo-vinylene carbonate groups or intermediates that beside an exo-vinylene carbonate group comprise additional functional groups for further manipulation or transfunctionalization, e.g., by dimerization, acrylation or the like.

Due to the unfavorable toxicology classification of low molecular isocyanates, it is an object of the present invention to provide alternative precursors for isocyanate-free urethane-type polymers. It is a further object of the invention that the precursors overcome the above shortcomings.

This object is solved by providing a compound containing both one or more urethane group(s) and one or more exo-vinylene carbonate groups (hereinafter also referred to as "exo-VC").

In a first aspect, the invention provides a compound of formula (I) 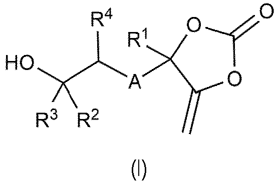 wherein
R¹ is H or methyl,
R² and R³ are independently selected from H, C₁-C₄ alkyl, cycloalkyl and phenyl-C₁-C₄-alkyl, or
R² and R³, together with the carbon atom to which they are attached, form a C₃-C₆ carbocycle,
R⁴ is H or C₁-C₄ alkyl, and
A is C₂-C₇-alkylene.

Depending on the substitution pattern, the compound of formula (I) can have one or more stereogenic centers. Herein, formula (I) is intended to denote all stereoisomers of the compound of formula (I).

The inventive compounds of formula (I) are suitable precursors for polyisocyanate-adducts and crosslinkers for obtaining isocyanate-free urethane-type polymers. Due to the presence of the hydroxyl group, the compounds of formula (I) give access to further functionalization, e.g., by reaction with an at least difunctional compound.

Quite surprisingly, despite the reactivity of the exo-VC group towards hydroxyl groups and the presence of a hydroxyl group in the molecule of the compound of formula (I), the compound of formula (I) is stable as such. This is believed to be due to the exo-VC group and the hydroxyl group being spaced apart via the moiety --CR²R³-CR⁴-A-- with A being C₂-C₇-alkylene. The -CR²R³-CR⁴-A- moiety ensures that the compound of formula (I) does not undergo intramolecular reaction between the hydroxyl group and the exo-VC group. In other words, "back biting" is avoided in the compound of formula (I).

Preferably, in formula (I),
R¹ is H or methyl,
R² and R³ are independently selected from H, C₁-C₄ alkyl, cycloalkyl and phenyl-C₁-C₄-alkyl,
R⁴ is H or C₁-C₄ alkyl, and
A is C₂-C₄-alkylene.

In an especially preferred compound of formula (I), R¹, R² and R³ are methyl, R⁴ is H, and A is -(CH₂)₂. This conforms with the compound of formula (I) being

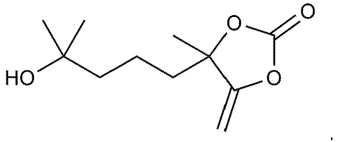

The invention further relates to a method for preparing a compound of formula (I), comprising hydrating a compound of formula (II) 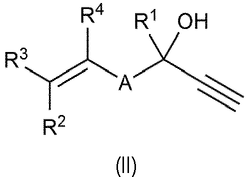 wherein A, R¹, R², R³ and R⁴ are as defined above.
to obtain a compound of formula (III) 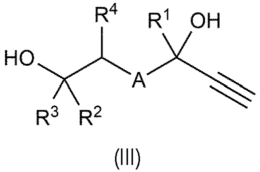
wherein A, R¹, R², R³ and R⁴ are as defined above,
and reacting the compound of formula (III) with carbon dioxide to obtain the compound of formula (I).

Hydration of the compound of formula (II) to obtain the compound of formula (III) is suitably carried out in an aqueous solution. Suitably, the hydration reaction is carried out at room temperature. Preferably, the hydration reaction is acid-catalyzed. Suitable acids include mineral acids such as sulfuric acid. For example, the hydration reaction is carried out using a 35 wt.-% aqueous solution of H₂SO₄. Suitable reaction conditions are also described in GB 2515128 A.

Reacting the compound of formula (III) with carbon dioxide to obtain the compound of formula (I) is suitably carried out at reaction temperatures in the range of from 30 to 200 °C, preferably 50 to 130 °C. Suitable reaction conditions are also described in DE 1 098 953.

Reacting the compound of formula (III) with carbon dioxide to obtain the compound of formula (I) is suitably carried out at reaction pressures are in the range of from 1 to 100 bar, preferably 3 to 50 bar of carbon dioxide. Reacting the compound of formula (III) with carbon dioxide to obtain the compound of formula (I) is usually carried out in a solvent such as methanol, tetrahydrofuran, 2-methyltetrahydrofuran, 1,4-dioxane, γ-valerolactone, acetonitrile, dichloromethane, and supercritical CO₂. At least a part of the solvent may be replaced by carbon dioxide (CO₂) at moderate pressures (tens of bars), a situation hereinafter referred to as "CO₂ expanded solvent". Suitably, at least 50 vol.-%, or at least 60 vol.-%, or at least 70 vol.-%, or at least 80 vol.-% of the solvent may be replaced by CO₂.

The reaction of the compound of formula (III) with carbon dioxide to obtain the compound of formula (I) is usually catalyzed.

Suitable catalysts include transition metal catalysts containing, for example, zinc, silver, copper, gold, palladium or platinum as the active metal, e.g. silver salts such as silver acetate, silver carbonate; copper(II) salts such as copper acetate or copper(I) halides such as Cui, CuBr, CuCI; and palladium(0) catalysts.

The transition metal compounds may be used in combination with an organic amine, an organic phosphine or a mixture of the organic phosphine with an ammonium salt. The organic amine may be selected from a tri-C₁-C₆-alkylamine such as triethylamine or an amidine base such as 1,5-diazabicyclo[4.3.0]non-5-ene (DBN) or 1,8-diazabicyclo[5.4.0]undec-7-ene (DBU). The organic phosphine may be selected from trialkylphosphines or triarylphosphines such as tributylphosphine and triphenylphosphine. The ammonium salt may be selected from tri-C₁-C₆-alkylammonium halides or tetra-C₁-C₆-alkylammonium halides.

Further suitable catalysts can be organic phosphines as such, e.g. trialkylphosphines or triarylphosphines such as tributylphosphine or triphenylphosphine, as well as sterically hindered carbenes, e.g. 1,3-substituted 2,3-dihydroimidazol-2-ylidene compounds such as 1,3-diisopropyl-2,3-dihydro-4,5-imidazole-2-ylidene compounds. e.g. 1,3-substituted 2,3-dihydroimidazol-2-ylidene compounds such as 1,3-diisopropyl-2,3-dihydro-4,5-imidazol-2-ylidene or their CO₂ adducts, as well as combinations thereof with the aforementioned phosphines.

Advantageously, the inventive process is devoid of a step requiring dedicated acetylene handling. Furthermore, it is a simple 3-step process performed at ambient reaction conditions resulting in high yields.

Preferably, in the compound of formula (II), R¹, R² and R³ are methyl, R⁴ is H, and A is -(CH₂)₂. In this case, the compound of formula (II) is dehydrolinalool (DHL) which is advantageous as DHL is a readily available large volume industrial product.

Typically, in large scale, DHL is produced starting by ethynylation of 6-methyl-5-hepten-2-one.

The compound of formula (I) comprises two reactive groups, namely the hydroxyl group and the exo-vinylene carbonate group. Thus, the compound of formula (I) may not act as crosslinking agent, e.g. in subsequent polymerization reactions. For obtaining a compound having crosslinking properties in subsequent polymerization reactions, the compound of formula (I) is therefore to be reacted to yield a compound having two or more reactive groups, e.g. by reaction with a polyisocyanate. This yields a precursor for urethane compounds containing exo-vinylene carbonate groups. Such urethane compounds containing exo-vinylene carbonate groups may be converted to polyurethane-hybrids after curing with a nucleophile.

In a second aspect, the invention relates to a compound of formula (IV): 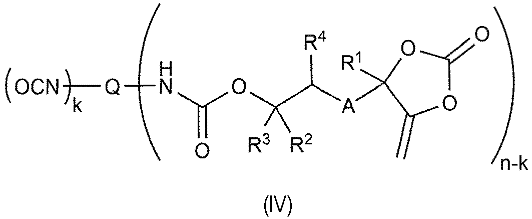 wherein
Q is the residue of an n functional polyisocyanate,
n is from 1.5 to 3, preferably 2 or 3,
k is from 0 to n-1,
R¹ is H or methyl,
R² and R³ are independently selected from H, C₁-C₄ alkyl, cycloalkyl and phenyl-C₁-C₄-alkyl, or
R² and R³, together with the carbon atom to which they are attached, form a C₃-C₆ carbocycle,
R⁴ is H or C₁-C₄ alkyl, and
A is C₂-C₇-alkylene.

A "residue of an n functional polyisocyanate" is a polyisocyanate with n isocyanate groups from which all isocyanate groups are removed.

Preferably, R¹, R² and R³ are methyl, R⁴ is H, and A is -(CH₂)₂.

The term "polyisocyanate" includes isocyanate-functional materials that generally include at least two isocyanate groups. Polyisocyanates include diisocyanates (materials with two isocyanate groups) and higher polyisocyanates such as triisocyanates (materials with three isocyanate groups), tetraisocyanates (materials with four isocyanate groups), and the like. Where polyisocyanates are applied as technical mixtures, the number of isocyanate groups n is given as the average number of isocyanate groups per molecule in the technical mixture.

The n functional polyisocyanate may be a monomeric polyisocyanate or oligomeric polyisocyanate.

Monomeric polyisocyanates are usually diisocyanates. Diisocyanates may be generally described by the structure OCN-Z-NCO, where the Z group may be an aliphatic group, an aromatic group, or a group containing a combination of aromatic and aliphatic groups. In some embodiments, it is preferable to use a monomeric aliphatic isocyanate, such as pentamethylenediisocyanate (PDI), hexamethylenedisocyanate (HDI), dicyclohexylmethane-4,4'-diisocyanate (H12MDI), isophorone diisocyanate (IPDI), dimeryl diisocyanate (DDI). The term "aliphatic" refers to the carbon atoms to which the NCO groups of the monomer are bonded, i.e. the compound molecule may perfectly well contain aromatic rings, which do not then of course carry NCO groups. In some embodiments, it is preferable to use an n functional monomeric aromatic isocyanate, such as toluene diisocyanate (TDI), 4,4'-methylene diphenyl diisocyanate (MDI), naphthalene diisocyanate (NDI), and oligomers, derivatives, or combinations of these isocyanates.

Oligomerization of isocyanates is a long-known, generally accepted method of modifying low molecular weight isocyanates, which are usually difunctional, in order to obtain products with advantageous application properties.

Higher functional polyisocyanates, i.e. n functional oligomeric polyisocyanates, may also be used. Such n functional oligomeric polyisocyanates include, but are not limited to, polyisocyanates, such as those produced from uretdiones, isocyanurates, biurets, allophanates, oxadiazinediones, iminooxadiazinediones, uretoneimines, carbodiimides, and the like. Particularly important procedures are so-called dimerization to form uretdione structures and so-called trimerization to form isocyanurate structures. In addition to the last-mentioned trimers, isomeric, i.e. also trimeric products with an iminooxadiazindione structure can be obtained.

Uretdione diisocyanates are cyclic dimerization products of diisocyanates. Preference is given to uretdione diisocyanates with aromatically, aliphatically and/or cycloaliphatically attached isocyanate groups, more preferably aliphatically and/or cycloaliphatically attached, and in particular those derived from hexamethylene diisocyanate or isophorone diisocyanate.

Regarding polyisocyanates having isocyanurate groups, preference is given to those derived from aromatic, aliphatic and/or cycloaliphatic diisocyanates. Particular preference is given to the corresponding aliphatic and/or cycloaliphatic isocyanatoisocyanurates and in particular to those based on hexamethylene diisocyanate and isophorone diisocyanate. These present isocyanurates are, in particular, tris-isocyanatoalkyl and/or tris-isocyanatocycloalkyl isocyanurates, which are cyclic trimers of the diisocyanates, or are mixtures with their higher homologs containing more than one isocyanurate ring. The isocyanatoisocyanurates generally have an NCO content of 10 to 30 wt.-%, in particular 15 to 25 wt.-%, and an average NCO functionality of 2.6 to 8.

Regarding polyisocyanates having biuret groups, preference is given to those having aromatically, cycloaliphatically or aliphatically attached, preferably cycloaliphatically or aliphatically attached, isocyanate groups, especially tris(6-isocyanatohexyl)biuret or mixtures with higher homologs thereof. These polyisocyanates having biuret groups generally have an NCO content of 18 to 22 wt.-% and an average NCO functionality of 2.8 to 4.5.

Regarding polyisocyanates having urethane and/or allophanate groups, preference is given to those having aromatically, aliphatically or cycloaliphatically attached, preferably aliphatically or cycloaliphatically attached, isocyanate groups. They may be obtained, for example, by reacting excess amounts of hexamethylene diisocyanate or of isophorone diisocyanate with mono- or polyhydric alcohols, for example methanol, ethanol, iso-propanol, n-propanol, n-butanol, iso-butanol, sec-butanol, tert-butanol, n-hexanol, n-heptanol, n-octanol, n-decanol, n-dodecanol (lauryl alcohol), 2-ethylhexanol, n-pentanol, stearyl alcohol, cetyl alcohol, lauryl alcohol, ethylene glycol monomethyl ether, ethylene glycol monoethyl ether, propane-1,3-diol monomethyl ether, cyclopentanol, cyclohexanol, cyclooctanol, cyclododecanol, trimethylolpropane, neopentyl glycol, pentaerythritol, butane-1,4-diol, hexane-1,6-diol, propane-1,3-diol, 2-ethylpropane-1,3-diol, 2-methylpropane-1,3-diol, ethylene glycol, diethylene glycol, triethylene glycol, tetraethylene glycol, pentaethylene glycol, glycerol, 1,2-dihydroxypropane, 2,2-dimethylethane-1,2-diol, butane-1,2-diol, butane-1,4-diol, 3-methylpentane-1,5-diol, 2-ethylhexane-1,3-diol, 2,4-diethyloctane-1,3-diol, hydroxypivalic acid neopentyl glycol ester, ditrimethylolpropane, dipentaerythritol, 2,2-bis(4-hydroxycyclohexyl)propane, cyclohexane-1,1-, -1,2-, - 1,3- and -1,4-dimethanol, cyclohexane-1,2-, -1,3- or -1,4-diol, or mixtures thereof. These polyisocyanates having urethane and/or allophanate groups generally have an NCO content of 12 to 20 wt.-% and an average NCO functionality of 2.5 to 4.5.

Regarding polyisocyanates having oxadiazinetrione groups, preference is given to those derived from hexamethylene diisocyanate or isophorone diisocyanate. Polyisocyanates of this kind comprising oxadiazinetrione groups are obtainable from diisocyanate and carbon dioxide.

Regarding polyisocyanates having iminooxadiazinedione groups, preference is given to those derived from hexamethylene diisocyanate or isophorone diisocyanate. Polyisocyanates of this kind comprising iminooxadiazinedione groups are preparable from diisocyanates by means of specific catalysts.

Suitable commercially available polyisocyanates include hexamethylenedisocyanate (HDI), isophorone diisocyanate (IPDI), members of the BASONAT series (available from BASF), members of the VESTANAT series (available from Evonik), members of the WANNATE series (available from Vencorex und Wanhua), and members of the DESMODUR and MONDUR series (available from Covestro AG).

Specifically, Q may be the residue of
an n functional monomeric polyisocyanate which is selected from
   - pentamethylenediisocyanate (PDI),
   - hexamethylenedisocyanate (HDI),
   - dicyclohexylmethane-4,4'-diisocyanate (H12MDI),
   - isophorone diisocyanate (IPDI),
   - dimeryl diisocyanate (DDI),
   - toluene diisocyanate (TDI),
   - 4,4'-methylene diphenyl diisocyanate (MDI),
   - naphthalene diisocyanate (NDI),
   - m-xylylenediisocyanate (XDI),
      or
an n functional oligomeric polyisocyanate which is selected from
   - oligomeric MDI (also referred to as "polymeric MDI" (PMDI)),
   - polyisocyanates having uretdione groups,
   - polyisocyanates having isocyanurate groups,
   - polyisocyanates having biuret groups,
   - polyisocyanates having urethane groups or allophanate groups,
   - polyisocyanates comprising oxadiazinetrione groups,
   - uretonimine-modified polyisocyanates,
   - carbodiimide-modified polyisocyanates,
   - polyurethane-polyisocyanate prepolymers or polyurea-polyisocyanate prepolymers, preferably of linear or branched C₄-C₂₀-alkylene diisocyanates and/or cycloaliphatic diisocyanates having a total of 6 to 20 carbon atoms,
or combinations of these polyisocyanates.

Isophorone diisocyanate (IPDI) comprises two isocyanate groups with different reactivities. Upon reaction of isophorone diisocyanate (IPDI), e.g. with an isocyanate-reactive group, it may depend on the reaction conditions which of the isocyanate groups reacts first. The resulting reaction product may comprise different constitutional isomers. In the structural formulae herein, one possible constitution of the reaction product is shown. However, this is also intended to denote other possible constitutional isomers, or mixtures.

The invention further relates to a method for preparing the compound of formula (IV) as defined above, comprising reacting an n functional polyisocyanate of formula (V) 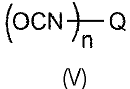 wherein
Q and n are as defined above,
with a compound of formula (I) as defined above to obtain the compound of formula (IV).

The method for preparing the compound of formula (IV) is suitably carried out at reaction temperatures in the range of from 20 to 150 °C.

The method for preparing the compound of formula (IV) is suitably carried out at a molar ratio of hydroxyl groups of the compound of formula (I) to isocyanate groups of the n functional polyisocyanate of formula (V) in the range of from 1 : 2 to 2 : 1.

The method for preparing the compound of formula (IV) is suitably carried out in a solvent, the solvent preferably being selected from tetrahydrofuran, γ-valerolactone, ethyl acetate, butyl acetate, acetone, methyl ethyl ketone, dichloromethane, toluene, and xylene.

The method for preparing the compound of formula (IV) is suitably carried out in the presence of a catalyst. Useful catalysts include metal-containing and non-metal-containing catalysts. Examples of the metal portion of the catalysts include tin, titanium, zirconium, lead, iron cobalt, antimony, manganese, bismuth and zinc compounds. Other suitable non-limiting examples of catalysts include chelates of various metals such as those which can be obtained from acetylacetone, benzoylacetone, trifluoroacetylacetone, ethyl acetoacetate, salicylaldehyde, cyclopentanone-2-carboxylate, acetylacetoneimine, bis-acetylaceone-alkylenediimines, salicylaldehydeimine, and the like, with the various metals such as Al, Be, Mg, Zn, Cd, Pb, Ti, Zr, Sn, As, Bi, Cr, Mo, Mn, Fe, Co, Ni, and metal oxide ions; alcoholates and phenolates of various metals such as Ti(OR)₄, Sn(OR)₄, Sn(OR)₂, Al(OR)₃, Bi(OR)₃ and the like, wherein R is alkyl or aryl of from 1 to 18 carbon atoms, and reaction products of alcoholates of various metals with carboxylic acids, beta-diketones, and 2-(N,N-dialkylamino)alkanols, such as well known chelates of titanium obtained by this or equivalent procedures.

Additional useful catalysts include organometallic derivatives of tetravalent tin, trivalent and pentavalent As, Sb, and Bi, and metal carbonyls of iron and cobalt; and combinations thereof. In one specific embodiment organotin compounds that are dialkyltin salts of carboxylic acids, can include the non-limiting examples of dibutyltin diacetate, dibutyltin dilaurate, dibutyltin maleate, dilauryltin diacetate, dioctyltin diacetate, dibutyltin-bis(4-methylaminobenzoate), dibutyltindilaurylmercaptide, dibutyltin-bis(6-methylaminocaproate), and the like, and combinations thereof. Similarly, there may be used trialkyltin hydroxide, dialkyltin oxide, dialkyltin dialkoxide, or dialkyltin dichloride and combinations thereof. Non-limiting examples of these compounds include trimethyltin hydroxide, tributyltin hydroxide, trioctyltin hydroxide, dibutyltin oxide, dioctyltin oxide, dilauryltin oxide, dibutyltin-bis(isopropoxide), dibutyltin-bis(2-dimethylaminopentylate), dibutyltin dichloride, dioctyltin dichloride, and the like, and combinations thereof.

After completion of the reaction, the catalyst is suitably removed by adsorption on an adsorbent selected from metal oxides and metal hydroxides, such as aluminum oxide, silica, clays, activated carbon, or combinations thereof.

While in the above method, the compound of formula (I) is reacted with an n functional polyisocyanate of formula (V) to yield the compound of formula (IV) as defined above, the reaction is carried out under the stringent workplace health and safety measures of a chemical manufacturing plant. The end user - in the use of the compound of formula (IV) as reaction partners for, for example, polyols in the production of synthetic resin systems or fixing systems - does not come into contact with toxic isocyanates.

The above-described compound of formula (IV) may have unreacted isocyanate groups. In a further application, said unreacted isocyanate groups may undergo a subsequent reaction with compounds having m isocyanate-reactive groups. For this purpose, the compound of formula (IV) preferably has one unreacted isocyanate group, i.e., in the compound of formula (IV), k is preferably 1.

This results in a compound of formula (VI) 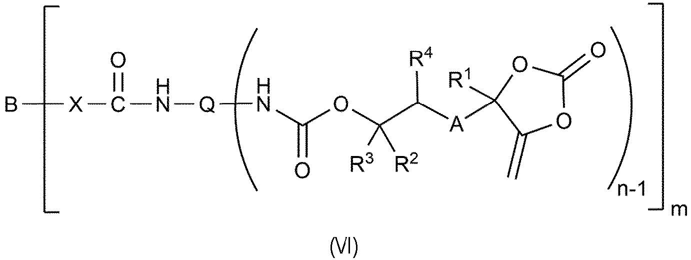 wherein
B is an m functional residue of a compound having m isocyanate-reactive groups,
X is O, S or NE, wherein E is H or C₁-C₄ alkyl,
m is from 2 to 8, preferably from 3 to 8,
Q is the residue of an n functional polyisocyanate,
n is from 1.5 to 3, preferably 2 to 3, more preferably 2,
R¹ is H or methyl,
R² and R³ are independently selected from H, C₁-C₄ alkyl, cycloalkyl and phenyl-C₁-C₄-alkyl, or
R² and R³, together with the carbon atom to which they are attached, form a C₃-C₆ carbocycle,
R⁴ is H or C₁-C₄ alkyl, and
A is C₂-C₇-alkylene.

A "residue of a compound having m isocyanate-reactive groups" is a compound having m isocyanate-reactive groups from which all isocyanate-reactive groups are removed.

Preferably, the compound having m isocyanate-reactive groups is selected from polyols, polyamines, alkanolamines, and polythiols, and mixtures thereof.

The polyol may be selected from alkanepolyols, intramolecular or intermolecular dehydration products of alkanepolyols, alkoxylation products of alkanepolyols, polyvinyl alcohols and acrylic polyols, polyesterpolyols, polyetherpolyols, and mixtures thereof.

Alkanepolyols and intramolecular or intermolecular dehydration products thereof, preferably C₃-C₈-alkanepolyols and intramolecular or intermolecular dehydration products thereof, include glycerin, 1,2,6-trihydroxyhexane, 1,2,3-butanetriol, 1,2,3-hexanetriol, trimethylolethane, trimethylolpropane, pentaerythritol, sorbitol, sorbitan, 1,2,3-cyclohexanetriol and polyglycerin.

Alkoxylation products of alkanepolyols are reaction products obtained from the reaction of any of the above-mentioned alkanepolyols with alkylene oxides. Suitable alkylene oxides are selected from C₂-C₄ alkylene oxides, preferably ethylene oxide and propylene oxide. The alkoxylation products of alkanepolyols may have an average degree of alkoxylation in the range of from 0.1 to 8, per hydroxyl group. Suitable commercially available alkoxylation products of alkanepolyols are selected from alkoxylated glycerol, alkoxylated pentaerytitol, alkoxylated di-pentaerythritol, alkoxylated sugar derivatives, alkoxylated trimethylolpropane, and alkoxylated trimethylolpropane dimer.

Polyvinyl alcohols suffice the following general formula (II)

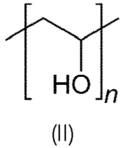

Suitable polyvinyl alcohols are those of formula (II) with n being selected such that the molecular weight of the polyvinyl alcohol is in the range of from 13000 to 130000.

Acrylic polyols (hydroxyl functional acrylic polymer) include copolymers of hydroxyethyl (meth)acrylate and other vinyl monomers.

Polyesterpolyols are known, for example, from Ullmanns Enzyklopädie der technischen Chemie, 4th Edition, Volume 19, pages 62 to 65. Polyesterpolyols may be obtained by reacting dihydric alcohols with dibasic carboxylic acids. For example, polyesterpolyols may be prepared from the above-mentioned polyols with polycarboxylic acids. Non-limiting examples of suitable polycarboxylic acids include phthalic acid, isophthalic acid, terephthalic acid, trimellitic acid, tetrahydrophthalic acid, adipic acid, succinic acid, glutaric acid, fumaric acid, and combinations thereof. Anhydrides of the above polycarboxylic acids can also be employed and are encompassed by the term "polycarboxylic acid". In addition, certain materials which react in a manner similar to acids to form polyesterpolyols can also be used. Non-limiting examples of such materials include lactones, such as caprolactone, propiolactone, and butyrolactone, and hydroxy acids, such as hydroxycaproic acid and dimethylol propionic acid. Moreover, as used herein, the polyesterpolyols can also include polyesterpolyols modified with fatty acids or glyceride oils of fatty acids. The polyesterpolyol can also be prepared by reacting an alkylene oxide, such as ethylene oxide, propylene oxide, and the like, and the glycidyl esters of versatic acid with methacrylic acid to form the corresponding ester. Suitable polyesterpolyols can also include polyester diols such as polycaprolactone diol. Non-limiting examples of commercially available polyesterpolyols include members of the LUPRAPHEN series (available from BASF), members of the DYNACOLL series (available from Evonik Industries) or members of the DESMOPHEN and BAYCOLL series (available from Covestro).

Polyetherpolyols are known per se may be prepared by self-polymerization of epoxides such as ethylene oxide, propylene oxide, butylene oxide or tetrahydrofuran, or by addition of these epoxides, preferably of ethylene oxide and propylene oxide, where appropriate mixed together or separately and consecutively, onto starter components having at least two reactive hydrogen atoms, such as water, ethylene glycol, propylene glycol, diethylene glycol, dipropylene glycol, glycerol, trimethylolpropane, pentaerythritol, sorbitol or sucrose. Representatives of the high molecular weight polyhydroxy compounds mentioned for use are listed for example in High Polymers, Vol. XVI, "Polyurethanes, Chemistry and Technology" (Saunders-Frisch, Interscience Publishers, New York, Vol. 1, 1962, pages 32-42).

The amino groups of the polyamine may be aliphatically-, cycloaliphatically-, araliphatically- or aromatically-bound.

The polyamine may be selected from
- aliphatic diamines or polyamines such as ethylene diamine, 1,2-propane diamine, 1,3-propane diamine, 1,4-butane diamine, 1,3-pentane diamine, 1,5-pentane diamine, 1,6-hexane diamine, 1,8-octane diamine, neopentane diamine, 1,10-decane diamine, 1,12-dodecane diamine, 2-methylpentane-1,5-diamine, N,N'-dimethyl-ethylene diamine, diethylene triamine, triethylene tetraamine, tetraethylene pentamine, pentaethylene hexamine, 2,2-dimethylpropylenediamine, trimethylhexamethylenediamine, 1-(3-aminopropyl)-3-aminopropane, 1,3-bis(3-aminopropyl)propane, 4-ethyl-4-methylamino-1-octylamine, N,N,N-tris-(2-aminoethyl)-amine, N,N,N'-tris-(2-aminoethyl)-ethylene diamine, polyethylene imines, polyvinylamines, polyallylamines, polylysines, iminobis-propylamine, N-(2-aminoethyl)-1,3-propane diamine, tetrapropylene pentamine, tripropylene tetramine, N,N-bis-(6-aminohexyl)-amine, N,N'-bis-(3-aminopropyl)-ethylene diamine, aminoethylethanolamine,
- cycloalkylenediamines or (cyclo)alkylenepolyamines such as cyclohexyldiamines such as 1,2-diaminocyclohexane, 1-methyl-2,4-diaminocyclohexane, 1-methyl-2,6-diaminocyclohexane, 1-amino-3-aminomethyl-3,5,5-trimethylcyclohexane (isophorone diamine), bis-(4-aminocyclohexyl)-methane, bis-(4-amino-3-methylcyclohexyl)-methane, 1,2-bis(aminomethyl)cyclohexane, 1,3-bis(aminomethyl)cyclohexane, 1,4-bis(aminomethyl)cyclohexane, 2,5-bisaminomethyl tetrahydrofuran, 4,4'-diamino-dicyclohexylmethane, 3,3'-dimethyl-4,4'-diamino-dicyclohexylmethane, 3,3',5,5'-tetramethyl-4,4'-diaminodicyclohexylmethane, N-cyclohexylpropylene-1,3-diamine, 4,8-diamino-tricyclo[5.2.1.0]decane, norbornandiamine, menthanediamine, menthenediamine,
- heterocyclic diamines or polyamines such as piperazine, 2,5-dimethyl piperazine, N-(2-piperazinoethyl) ethylene diamine, N,N'-bis-(2-aminoethyl)-piperazine, N-[N-(2-aminoethyl)-2-amino-ethyl]-N'-(2-aminoethyl)-piperazine, N-(2-aminoethyl)-N'-(2-piperazinoethyl)-ethylene diamine, N,N-bis-(2-aminoethyl)-N-(2-piperazinoethyl)-amine, N,N-bis-(2-piperazinoethyl)-amine,
- hydrazine, aminoacid hydrazides, hydrazides of semicarbazido carboxylic acids, bis-hydrazides, bis-semicarbazides,
- guanidine,
- aromatic diamines or polyamines such as melamine, 3,3'-diaminobenzidine, 2,4,6-triaminopyrimidine, 2,4-bis-(4'-aminobenzyl)-aniline, diethyl-toluene diamine isomers, toluene diamine isomers, xylylene diamine isomers, 1,2-phenylene diamine, 1,3-phenylene diamine, 1,4-phenylene diamine, methylene-bis-(phenylamine) isomers such as 4,4'-diamino-diphenylmethane or 4,4'-diaminodiphenylsulfone, 2,5-bisaminomethyl furan, 1,5-naphthalene diamine, aniline, alkyl anilines, toluidine, t-butyl-toluene diamine isomers, methylene-bis-(o-dichloroaniline) (MOCA), 2,4-diaminoalkylbenzene isomers having 8 to 15 carbon atoms in the alkyl chain,
- polyetheramines (alkylene diamines or alkylene polyamines comprising ether groups) such as difunctional and trifunctional primary polyetheramines based on polypropylene glycol, polyethylene glycol, polybutylene oxide, poly-(1,4-butanediol), polytetrahydrofuran (poly-THF) or polypentylene oxide, e.g. 4,7,10-trioxatridecan-1,3-diamine, 4,7,10-trioxatridecan-1,13-diamine, 1,8-diamino-3,6-dioxaoctane (XTJ-504, Huntsman), 1,10-diamino-4,7-dioxadecane (XTJ-590, Huntsman), 1,12-diamino-4,9-dioxadodecane (BASF SE), 1,3-diamino-4,7,10-trioxatridecane (BASF SE), primary polyetheramines based on polypropylene glycol with an average molecular weight of 230, such as Polyetheramine D 230 (BASF SE) or Jeffamine^{®} D 230 (Huntsman), difunctional, primary polyetheramines based on polypropylene glycol with an average molecular weight of 400, e.g. Polyetheramine D 400 (BASF SE) or Jeffamine^{®} XTJ 582 (Huntsman), difunctional, primary polyetheramines based on polypropylene glycol with an average molecular weight of 2000, e.g. Polyetheramine D 2000 (BASF SE) or Jeffamine^{®} XTJ 582 (Huntsman), difunctional, primary polyetheramines based on polypropylene glycol with an average molecular weight of 2000, e.g. Polyetheramine D 2000 (BASF SE) or Jeffamine^{®} XTJ 582 (Huntsman), e.g. Polyetheramine D 2000 (BASF SE), Jeffamine^{®} D2000 or Jeffamine^{®} XTJ 578 (both from Huntsman), difunctional, primary polyetheramines based on propylene oxide with an average molecular weight of 4000, e.g. Polyetheramine D 4000 (BASF SE), trifunctional, primary polyetheramines prepared by reaction of propylene oxide with trimethylolpropane, followed by amination of the terminal OH groups with an average molecular weight of 403, e.g. Polyetheramine T 403 (BASF SE) or Jeffamine^{®} T 403 (Huntsman), trifunctional, primary polyetheramines prepared by reaction of propylene oxide with glycerol, followed by amination of the terminal OH groups with an average molecular weight of 5000 (Huntsman), trifunctional, primary polyetheramine prepared by reaction of propylene oxide with glycerol, followed by amination of the terminal OH groups with an average molecular weight of 5000, such as Polyetheramine T 5000 (BASF SE) or Jeffamine^{®} T 5000 (Huntsman), aliphatic polyetheramines, which are composed of a polyethylene glycol grafted with propylene oxide and have an average molecular weight of 600, such as Jeffamine^{®} ED-600 or Jeffamine^{®} XTJ-501 (Huntsman), aliphatic polyetheramines which are composed of a polyethylene glycol grafted with propylene oxide and have an average molecular weight of 900, such as Jeffamine^{®} ED-900 (Huntsman), aliphatic polyetheramines which are composed of a polyethylene glycol grafted with propylene oxide and have an average molecular weight of 2000, e.g. Jeffamine^{®} ED-2003 (Huntsman), difunctional, primary polyether amines produced by amination of a diethylene glycol grafted with propylene oxide with an average molecular weight of 220, e.g. Jeffamine^{®} HK-511 (Huntsman), aliphatic polyether amines based on a copolymer of poly(tetramethylene ether glycol) and polypropylene glycol with an average molecular weight of 1000 (Huntsman), aliphatic polyether amines based on a copolymer of poly(tetramethylene ether glycol) and polypropylene glycol with an average molecular weight of 1000, such as Jeffamine^{®} XTJ-542 (Huntsman), aliphatic polyether amines based on a copolymer of poly(tetramethylene ether glycol) and polypropylene glycol with an average molecular weight of 1900, such as Jeffamine^{®} XTJ-542 (Huntsman), aliphatic polyether amines based on a copolymer of poly(tetramethylene ether glycol) and polypropylene glycol with an average molecular weight of 1900, such as Jeffamine^{®} XTJ-542 (Huntsman), polyether triamines based on an at least trivalent alcohol grafted with butylene oxide with an average molecular weight of 400, e.g. Jeffamine^{®} XTJ-566 (Huntsman), aliphatic polyether amines produced by amination of alcohols grafted with butylene oxide with an average molecular weight of 219, e.g. Jeffamine^{®} XTJ-568 (Huntsman), polyetheramines based on pentaerythritol and propylene oxide with an average molecular weight of 600, e.g. Jeffamine^{®} XTJ-616 (Huntsman), polyetheramines based on triethylene glycol with an average molecular weight of 148, e.g. Jeffamine^{®} EDR-148 (Huntsman), difunctional, primary polyether amines produced by amination of an ethylene glycol grafted with propylene oxide with an average molecular weight of 176, e.g. Jeffamine^{®} EDR-176 (Huntsman) and polyether amines prepared by amination of polytetrahydrofuran (Poly-THF) with an average molecular weight of 250, e.g. PolyTHF-Amin 350 (BASF SE),
- amino acids with two amino groups such as lysin, ornithin,
- polyamidoamines (amidopolyamines) which are obtainable by the reaction of dimeric fatty acids (e.g. dimeric linoleic acid) with low molecular weight polyamines such as diethylenetriamine, 1-(3-aminopropyl)-3-aminopropane or triethylenetetramine or other diamines such as the aliphatic or cycloaliphatic diamines,
- adducts which are obtainable by reacting amines, in particular diamines, with an admixture of epoxy resin or reactive diluent, preferably using adducts in which about 5 to 20 % of the epoxy groups have been reacted with amines, in particular diamines,
- Mannich bases which are obtained, for example, by condensation of polyamines, preferably diethylenetriamine, triethylenetetramine, isophorone diamine, 2,2,4- or 2,4,4-trimethylhexamethylenediamine, 1,3- and 1,4-bis(aminomethyl)cyclohexane with aldehydes, preferably formaldehyde and mono- or polyhydric phenols with at least one aldehyde-reactive core site, e.g. the various cresols and xylenols, p-tert-butylphenol, resorcinol, 4,4'-dihydroxydiphenylmethane, 4,4'-dihydroxydiphenyl-2,2-propane, preferably phenol.
- and mixtures thereof.

The polyamine may be silane-functionalized.

The alkanolamine may be selected from monoalkanolamines, dialkanolamines, trialkanolamines, tetraalkanolamines, or combinations thereof.

Examples of suitable monoalkanolamines include methylethanolamine, ethylethanolamine, methylisopropanolamine, ethylisopropanolamine, methyl-2-hydroxybutylamine, phenylethanolamine, ethanolamine, isopropanolamine, and combinations thereof.

Suitable dialkanolamines include dialkanolamines which include two hydroxy-substituted C₁-C₁₂ alkyl groups (e.g., two hydroxy-substituted C₁-C₈ alkyl groups, or two hydroxy-substituted C₁-C₆ alkyl groups). The two hydroxy-substituted alkyl groups can be branched or linear, and can be of identical or different chemical composition. Examples of suitable dialkanolamines include diethanolamine, diisopropanolamine, ethanolisopropanolamine, ethanol-2-hydroxybutylamine, isopropanol-2-hydroxybutylamine, isopropanol-2-hydroxyhexylamine, ethanol-2-hydroxyhexylamine, and combinations thereof.

Suitable trialkanolamines include trialkanolamines which include three hydroxy-substituted C₁-C₁₂ alkyl groups (e.g., three hydroxy-substituted C₁-C₈ alkyl groups, or three hydroxy-substituted C₁-C₆ alkyl groups). The three hydroxy-substituted alkyl groups can be branched or linear, and can be of identical or different chemical composition. Examples of suitable trialkanolamines include triisopropanolamine (TIPA), triethanolamine, N,N-bis(2-hydroxyethyl)-N-(2-hydroxypropyl)amine (DEIPA). N,N-bis(2-hydroxypropyl)-N-(hydroxyethyl)amine (EDIPA), tris(2-hydroxybutyl)amine, hydroxyethyl di(hydroxypropyl)amine, hydroxypropyl di(hydroxyethyl)amine, tri(hydroxypropyl)amine, hydroxyethyl di(hydroxy-n-butyl)amine, hydroxybutyl di(hydroxypropyl)amine, and combinations thereof.

Exemplary tetraalkanolamines include four hydroxy-substituted C₁-C₁₂ alkyl groups (e.g., four hydroxy-substituted C₁-C₈ alkyl groups, or four hydroxy-substituted C₁-C₆ alkyl groups).

The polythiol may be selected from glycol-bis(2-mercaptoacetate), glycol-bis(3-mercaptopropionate), 1,2-propylene glycol-bis(2-mercaptoacetate), 1,2-propylene glycol-bis(3-mercaptopropionate), 1,3-propylene glycol-bis(2-mercaptoacetate), 1,3-propylene glycol-bis(3-mercaptopropionate), tris(hydroxymethyl)methane-tris(2-mercaptoacetate), tris(hydroxymethyl)methane-tris(3-mercaptopropionate), 1,1,1-tris(hydroxymethyl)ethane-tris(2-mercaptoacetate), 1,1,1-tris(hydroxymethyl)ethane-tris(3-mercaptopropionate), 1,1,1-trimethylolpropane-tris(2-mercaptoacetate), ethoxylated 1,1,1-trimethylolpropane-tris(2-mercaptoacetate), propoxylated 1,1,1-trimethylolpropane-tris(2-mercaptoacetate), 1,1,1-trimethylol propane-tris(3-mercaptopropionate), ethoxylated 1,1,1 -trimethylolpropane-tris(3-mercaptopropionate), propoxylated trimethylolpropane-tris(3-mercaptopropionate), 1,1,1-trimethylolpropane-tris(3-mercaptobutyrate), pentaerythritol-tris(2-mercaptoacetate), pentaerythritol-tetrakis(2-mercaptoacetate), pentaerythritol-tris(3-mercaptopropionate), pentaerythritol-tetrakis(3-mercaptopropionate), pentaerythritol-tris(3-mercaptobutyrate), pentaerythritol-tetrakis(3-mercaptobutyrate), Capcure^{®} 3-800, GPM-800 (Gabriel Performance Products), Capcure^{®} LOF, GPM-800LO (Huntsman), KarenzMT PE-1 (Showa Denko), 2-ethylhexylthioglycolate, iso-octylthioglycolate, di(n-butyl)thiodiglycolate, glycol-di-3-mercaptopropionate, 1,6-hexanedithiol, 3,6-dioxa-1,8-octanedithiol, and tetra(ethylene glycol)dithiol.

Preferably, in the compound of formula (VI) above, X is O. Although any of the above-mentioned polyols, polyamines, alkanolamines, and polythiols may react with the compound of formula (IV) to yield the compound of formula (VI), preference is given to polyols. While polyamines, alkanolamines, and polythiols may react at least partially with the exo-VC group besides the isocyanate group of the compound of formula (IV), polyols preferably react with the isocyanate group(s) while the exo-VC group advantageously remains unreacted.

The invention further relates to a method for preparing the compound of formula (VI) as defined above, comprising reacting a compound having m isocyanate-reactive groups of formula (VII) 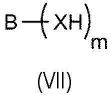 wherein m, B and X are as defined above,
with a compound of formula (IV) as defined above with k = 1 to obtain the compound of formula (VI).

The method for preparing the compound of formula (VI) is suitably carried out at reaction temperatures in the range of from 20 to 150 °C.

The method for preparing the compound of formula (VI) is suitably carried out at a molar ratio of isocyanate groups in the compound of formula (IV) to the isocyanate-reactive groups in the compound having m isocyanate-reactive groups of formula (VII) in the range of from 1 : 2 to 2 : 1.

The method for preparing the compound of formula (VI) is suitably carried out in a solvent, the solvent preferably being selected from tetrahydrofuran, γ-valerolactone, ethyl acetate, butyl acetate, acetone, methyl ethyl ketone, dichloromethane, toluene, and xylene.

The method for preparing the compound of formula (VI) is suitably carried out in the presence of a catalyst, the catalyst preferably being selected from dibutyltin dilaurate (DBTL). After completion of the reaction, the catalyst is suitably removed by adsorption on an adsorbent selected from metal oxides and metal hydroxides, such as aluminum oxide, silica, clays, activated carbon, or combinations thereof.

In a further aspect of the invention, further reactive groups may be introduced into compounds of formula (IV) as described above by introducing polymerizable groups which may subsequently be polymerized.

This results in a radically polymerizable compound of formula (VIII) 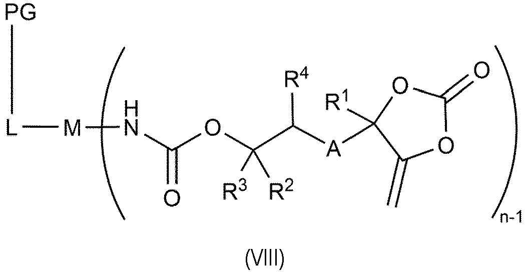 wherein
M is 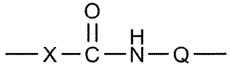 or a chemical bond,
PG is a radically polymerizable group,
L is a linker,
X is O, S or NE, wherein E is H or C₁-C₄ alkyl,
Q is the residue of an n functional polyisocyanate,
n is from 1.5 to 3, preferably 2,
R¹ is H or methyl,
R² and R³ are independently selected from H, C₁-C₄ alkyl, cycloalkyl and phenyl-C₁-C₄-alkyl, or
R² and R³, together with the carbon atom to which they are attached, form a C₃-C₆ carbocycle,
R⁴ is H or C₁-C₄ alkyl, and
A is C₂-C₇-alkylene.

In one aspect, the term "linker" denotes a divalent molecular entity that connects two parts of a compound, e.g. PG and X in the context of the compound of formula (VIII). Suitably, the "divalent molecular entity" is a chain of successive carbon atoms. Preferably, the chain has 2 to 12 carbon atoms, in particular 2 to 6 carbon atoms. The chain is optionally substituted with 1 to 5 identical or different substituents, independently of one another selected from C₁-C₄ alkyl, cycloalkyl and phenyl-C₁-C₄-alkyl.

The linker may also be a covalent bond meaning that in this case, no molecular entity is present between the two parts of the compound to be connected. In the context of the compound of formula (VIII), the linker being a covalent bond refers to a situation in which PG and X are directly bound.

In an embodiment, M is

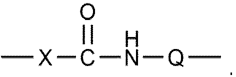

For example, PG may be selected from a (meth)acryloyloxy group and an allyl group. The resulting compound of formula (VIII) may be obtained by reacting the compound of formula (IV) with, e.g., a hydroxyalkyl (meth)acrylate such as hydroxymethyl (meth)acrylate or hydroxyethyl (meth)acrylate, or a C₃₊-alkenol such as allyl alcohol or isoprenol.

Herein, the designator "Cₓ" refers to a hydrocarbon including x carbon atoms; "Cₓ₊" refers to a hydrocarbon or mixture of hydrocarbons including x or greater carbon atoms.

In an embodiment, PG is an allyl group, L is a bond and X is O.

In a preferred embodiment, PG is a (meth)acryloyloxy group, L is C₂-C₆ alkylene, and X is O.

In an embodiment, M is a chemical bond. The resulting compound of formula (VIII) may be obtained by reacting the compound of formula (I) with, e.g., a (meth)acryloyloxyalkyl isocyanate, preferably (meth)acryloyloxyethyl isocyanate. In a preferred embodiment, PG is a (meth)acryloyloxy group and L is C₂-C₆ alkylene.

The radically polymerizable compound of formula (VIII) may be polymerized yielding a polymeric compound, comprising a polymeric backbone comprised of a plurality of backbone units, of the formula (VIII): 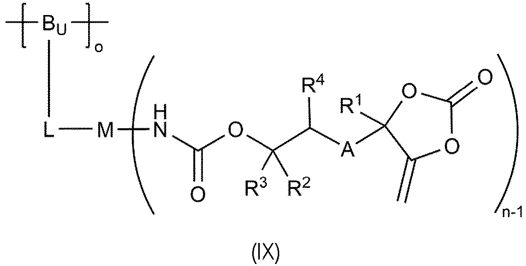 wherein
M is 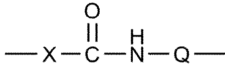 or a chemical bond,
Bu is a backbone unit within the polymeric backbone,
o is 2 to 50,
L is a linker,
X is O, S or NE, wherein E is H or C₁-C₄ alkyl,
Q is the residue of an n functional polyisocyanate,
n is from 1.5 to 3, preferably 2,
R¹ is H or methyl,
R² and R³ are independently selected from H, C₁-C₄ alkyl, cycloalkyl and phenyl-C₁-C₄-alkyl, or
R² and R³, together with the carbon atom to which they are attached, form a C₃-C₆ carbocycle,
R⁴ is H or C₁-C₄ alkyl, and
A is C₂-C₇-alkylene.

Suitable polymerization initiators for initiating polymerization of the radically polymerizable compound of formula (VIII) may be a peroxo-type initiator, an azo-type initiator or a redox initiator system.

Examples of a peroxo-type initiator include t-amyl peroxybenzoate, dibenzoyl peroxide, 2,2-bis(t-butylperoxy)butane, 1,1-bis(t-butylperoxy)-cyclohexane, 2,5-bis(t-butylperoxy)-2,5-dimethylhexane, 1,1-bis(t-butylperoxy)-3,3,5-trimethylcyclohexane, t-butyl hydroperoxide, t-butyl peracetate, t-butyl-peroxypivalate, t-amyl-peroxypivalate, t-butylperoxide, t-butyl peroxybenzoate, t-butylperoxy isopropyl carbonate, cumene hydroperoxide, cyclohexanone peroxide, dicumyl peroxide, lauroyl peroxide, 2,4-pentanedione peroxide, peracetic acid and potassium persulfate.

Examples of an azo-type initiator include 2,2'-azobisiso-butyronitrile (AIBN), 1,1'-azobis(cyclo-hexanecarbonitrile), 2,2'-azobis(2-methylbutyronitrile), 2-t-butylazo-2-cyanopropane and dimethyl-2,2'-azobis(2-methylpropionate).

Examples of redox initiator system are combinations of an oxidizing compound and a reducing compound. Examples of an oxidizing compound include the peroxo-type initiators listed above. Examples of a reducing compound include sulfur compounds with a low oxidation state such as alkali metal sulfites, for example potassium and/or sodium sulfite, alkali metal hydrogensulfites, for example potassium and/or sodium hydrogensulfite, alkali metal metabisulfites, for example potassium and/or sodium metabisulfite, formaldehyde sulfoxylates, for example potassium and/or sodium formaldehyde sulfoxylate, alkali metal salts, specifically potassium and/or sodium salts of aliphatic sulfinic acids and alkali metal hydrogensulfides, for example potassium and/or sodium hydrogensulfide, salts of polyvalent metals, such as iron(II) sulfate, iron(II) ammonium sulfate, iron(II) phosphate, ene diols such as dihydroxymaleic acid, benzoin and/or ascorbic acid, and reducing saccharides such as sorbose, glucose, fructose and/or dihydroxyacetone.

Polymerization of the radically polymerizable compound of formula (VIII) to obtain the polymeric compound of formula (IX) is suitably carried out at reaction temperatures in the range of from 60 to 200 °C.

Polymerization of the radically polymerizable compound of formula (VIII) to obtain the polymeric compound of formula (IX) is suitably carried out in a solvent.

Suitable solvents include aprotic solvents or protic solvents as well as any mixture of an aprotic and protic solvent.

Examples of aprotic solvents include aromatic hydrocarbons like toluene, xylenes, o-xylene or chlorobenzene; aliphatic hydrocarbons like cyclohexane, n-heptane or 1,2-dichloroethane; ketones like cyclohexanone, acetone, butanone or methyl amyl ketone; ethers like dioxane, monoglyme, 2-methyl-tetrahydrofuran or anisole; esters like ethyl acetate, propyl acetate, n-butyl acetate, methyl butyrate, ethyl butyrate, propyl butyrate, methyl propionate, ethyl propionate, propyl propionate, cyclohexyl acetate, 1-methoxy-2-propyl acetate, 2-methoxyethyl acetate, γ-butyrolactone or γ- or δ-valerolactone; amides like N,N-dimethylformamide, N,N-dimethylacetamide, N-methylpyrrolidone or N-ethylpyrrolidone; sulfoxides like dimethyl sulfoxide; or mixtures of two or more of these aprotic solvents.

Examples of protic solvents include alcohols like n-propanol, 2-propanol, n-butanol, 2-butanol, isobutanol, t-butanol, n-pentanol, 2-pentanol, benzyl alcohol, 1-methoxy-2-propanol or 1-methoxy-2-methyl-2-propanol; or mixtures of two or more of these protic solvents.

Preferred solvents are aromatic hydrocarbons like xylenes or o-xylol; alkyl esters like ethyl acetate, n-butyl acetate or 1-methoxy-2-propylacetate; alcohols like 2-propanol, 2-butanol, 2-pentanol or 1-methoxy-2-propanol; ketones like cyclohexanone or butanone; and any mixture of two or more thereof.

In an embodiment, an approach to convert a compound comprising two exo-VC groups (bis-functional compound) into a higher functional compound is provided. To this end, a compound comprising two exo-VC groups (bis-functional compound), e.g. a compound of formula (IV) with n being 2 and k being 0, may be reacted with an at least trivalent nucleophile, e.g. a triol or a triamine. Suitably, a molar excess of exo-VC groups over nucleophilic groups is employed. In this case, one of the exo-VC groups is "sacrificed" and thus no longer available for subsequent curing reactions. Due to the reaction with an at least trivalent nucleophile, the reaction yields a compound having crosslinking ability (multifunctional compound).

It has been shown that the inventive compounds of formula (I) are capable of reacting with nucleophiles such as primary amines, secondary amines and/or alcohols in various ways. This is summarized in the following scheme:

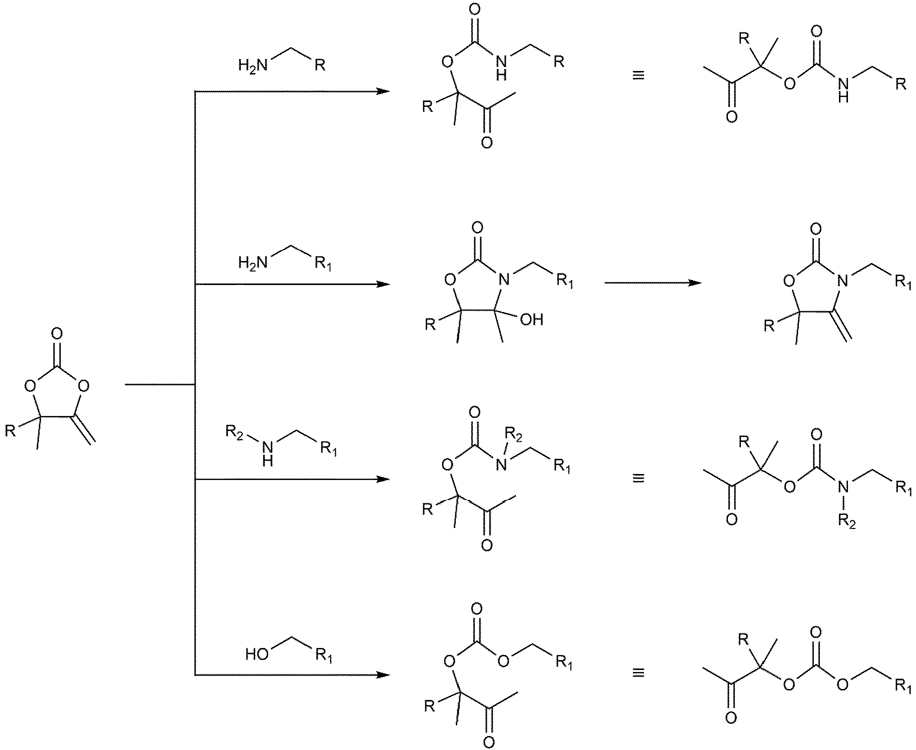

Reaction of the compounds of formula (I) with primary amines can primarily yield vinylene oxazolidinones. Reaction of the compounds of formula (I) with secondary amines can primarily yield carbamates. Reaction of the compounds of formula (I) with alcohols can primarily yield carbonates. Thus, the inventive compounds as described above are capable of reacting with polyols, polyamines, alkanolamines, and polythiols, and mixtures thereof and may therefore advantageously replace reaction with isocyanates.

The resulting polymers incorporate urethane bonds and bonds formed by the reaction of the exo-VC group with a nucleophile. Hence, depending on the nature of the nucleophile and the reaction conditions, the polymers can be polyurethane polymers or are mixed backbone polymers, which means that they incorporate more than one backbone bond type.

Finally, the invention further relates to a composition comprising a compound of any one of formulae (I), (IV), (VI), (VIII) and/or (IX) as described above.

The invention is illustrated by the examples that follow.

Thin-layer chromatography (TLC) was performed on precoated plates (silica gel 60, F₂₅₄). The plates were evaluated under UV light (254 nm) or by staining with basic KMnO₄ followed by heating.

For flash column chromatographic purification silica gel 60 (40 - 63 µm) was used. If not otherwise stated, cyclohexane and ethyl acetate were used as eluent system.

NMR spectroscopic spectra were recorded using multiple devices including Bruker Avance 300, Bruker Avance II 400, Bruker Avance Neo 400 and Bruker Avance III HD 300. ¹H NMR spectra were measured at 300 MHz or 400 MHz and ¹³C NMR spectra at 75 MHz or 100 MHz.

The chemical shifts are given in ppm, relative to the solvent signal, whereas coupling constants J are reported in Hz. The multiplicities found are abbreviated according to the standard notation. If not otherwise stated, the measurements were performed at 300 K.

For high-resolution mass spectroscopy (HRMS), multiple devices were used, including Thermo Scientific Exactive, Agilent 6545 LC/Q-TOF and Agilent 6546 LC/Q-TOF with ESI or APCI ionization.

IR spectrometry was performed on an Agilent Technologies Cary 630 FTIR using FTIR. The found peaks were classified according to their relative strength as weak (w), medium (m) and strong (s).

TGA and DSC measurements were performed on a STA409 from Netsch Gerätebau GmbH.

### Examples

### Methods

### I. Compound(s) of formula (I)

### Preparation of 2,6-dimethyloct-7-yne-2,6-diol

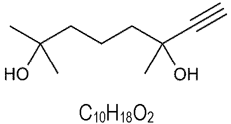

In a 250 mL round-bottomed flask, 90 mL of H₂O were mixed with 20 mL of conc. H₂SO₄ to obtain a 29% H₂SO₄ solution. To this, dehyrolinalool (40 mL, 231.2 mmol, 1.0 equiv.) were added in one portion, followed by vigorous stirring at room temperature for 6 days. A dark-brownish solution was obtained. The reaction mixture was extracted with diethylether (3 x 150 mL). The combined organic layers were washed with 3M NaOH (100 mL), saturated NaHCOs (100 mL), dried over Na₂SO₄ and concentrated in vacuum. A crude product was received as a brown oil. Vacuum distillation (1 mbar, 85-120 °C) afforded the product along with some starting material-containing mixed fractions. The product was obtained as a yellow oil in 40% yield (15.77 g, 92.6 mmol).

¹H NMR (300 MHz, CDCl₃): δ 2.92 (s, 1H), 2.40 (s, 1H), 1.84 (s, 1H), 1.68 - 1.46 (m, 6H), 1.45 (s, 3H), 1.19 (s, 6H).

¹³C{¹H} NMR (75 MHz, CDCl₃): δ 88.0, 71.4, 71.3, 67.9, 43.8, 43.7, 29.9, 29.3, 19.5.
GC MS (TG70eV-TG-for monomers 50-1000_9min): 3.10-3.15 min
GC MS (TG70eV-TG-for monomers 50-1000_15min): 3.10-3.15 min
R_{f} (isohexane/EtOAc 1:1) = 0.40 [KMnO₄]
HRMS (APCI): [m/z] calculated for C₁₀H₁₈O₂H⁺ ([M+H]⁺): 171.1380; found: 171.1380.

### Preparation of 4-(4-hydroxy-4-methylpentyl)-4-methyl-5-methylene-1,3-dioxolan-2-one

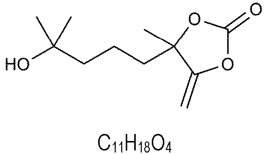

To a solution of 2,6-dimethyloct-7-yne-2,6-diol (15.2 g, 89.3 mmol) in CH₃CN (120 mL), DavePhos (1.76 g, 4.46 mmol) and AgOAc (0.75 g, 4.5 mmol) was added and the solution was charged to a steel autoclave under atmospheric conditions. The equipment was sealed, the reaction mixture was pressurized with CO₂ (20 bar) and stirred at room temperature. After 18 hours, CO₂ overpressure was carefully released. SiO₂ (30 g) was added to the reaction mixture and it was filtrated by glass filter, and then washed with CH₃CN. The solvent was removed under reduced pressure to give the product (17.4 g, 91%, GC: >99% pure). The purity can be improved by distillation (bp: 110-130 °C, 0.2 mbar).

¹H NMR (300 MHz, CDCl₃): δ 4.79 (d, *J* = 3.9 Hz, 1H), 4.27 (d, *J* = 3.9 Hz, 1H), 1.91 -1.79 (m, 1H), 1.77 -1.64 (m, 1H), 1.57 (s, 3H), 1.48 - 1.43 (m, 3H), 1.39 (s, 1H), 1.19 (s, 6H).

¹³C{¹H} NMR (75 MHz, CDCl₃): δ 157.7, 151.6, 87.3, 85.8, 70.8, 43.3, 40.8, 29.5, 26.4, 18.0.
R_{f} (isohexane/EtOAc; 1:1) = 0.32 [KMnO₄]
HRMS (APCI): [m/z] calculated for C₁₀H₁₈O₄NH₄⁺ ([M+NH₄]⁺): 232.1543; found: 232.1544.
Melting Point: ~60°C (±5 °C)

### II. Compound(s) of formula (IV)

### Preparation of bis(2-methyl-5-(4-methyl-5-methylene-2-oxo-1,3-dioxolan-4-yl)pentan-2-yl)hexane-1,6-diyldi-carbamate

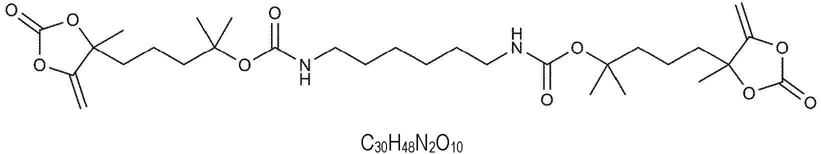

In a 10 mL vial, 4-(4-hydroxy-4-methylpentyl)-4-methyl-5-methylene-1,3-dioxolan-2-one (2.14 g, 9.99 mmol, 2.0 equiv.) was dissolved in THF (5 mL). While stirring at 50 °C, dibutyltin dilaurate (60 µL, 0.1 mmol, 2 mol-%) was added to the clear solution. Afterwards, hexamethylenedisocyanate (800 µL, 5.0 mmol, 1.0 equiv.) was added in one portion. Stirring was continued at 50 °C for 2 h, followed by addition of H₂O (10 mL). Then, while stirring, the reaction solution was allowed to cool to room temperature. The reaction solution was extracted with CH₂Cl₂ (3 x 30 mL). The combined organic layers were dried over Na₂SO₄ and concentrated in vacuum. The raw product was received as a slightly yellowish, medium-viscous oil in 99% solvent-corrected yield (2.95 g, 4.95 mmol, containing THF and CH₂Cl₂ as solvent impurities).

¹H NMR (300 MHz, CDCl₃): δ 4.79 (d, *J =* 3.9 Hz, 2H), 4.61 (t, *J =* 5.9 Hz, 2H), 4.26 (d, *J =* 3.9 Hz, 2H), 3.08 (q, *J =* 6.7 Hz, 4H), 1.83 - 1.67 (m, 8H), 1.57 (s, 6H), 1.51 - 1.40 (m, 6H), 1.39 (s, 12H), 1.31 (p, *J* = 3.5 Hz, 6H). ¹³C{¹H} NMR (75 MHz, CDCl₃): δ 157.8, 155.9, 151.6, 87.3, 85.7, 80.6, 40.6, 40.6, 30.1, 26.6, 26.5, 26.4, 17.7.
R_{f} (EtOAc) = 0.17 [KMnO₄]
HRMS (ESI): [m/z] calculated for C₃₀H₄₈N₂O₁₀Na⁺ ([M+Na]⁺): 619.3201; found: 619.3209.
HRMS (ESI): [m/z] calculated for C₃₀H₄₈N₂O₁₀Cl⁻ ([M+Cl]⁻): 631.3003; found: 631.3020.

### Preparation of 2-methyl-5-(4-methyl-5-methylene-2-oxo-1,3-dioxolan-4-yl)pentan-2-yl (3,3,5-trimethyl-5-(((((2-methyl-5-(4-methyl-5-methylene-2-oxo-1,3-dioxolan-4-yl)pentan-2-yl)oxy)carbonyl)amino)methyl)cyclohexyl)carbamate

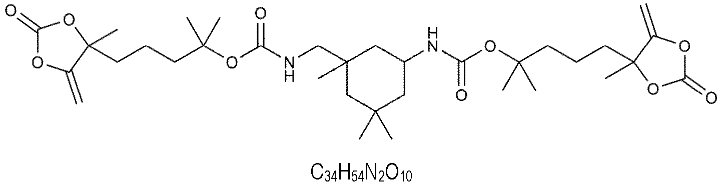

In a 10 mL vial, 4-(4-hydroxy-4-methylpentyl)-4-methyl-5-methylene-1,3-dioxolan-2-one (214 mg, 1.0 mmol, 2.0 equiv.) was dissolved in a solution of dibutyltin dilaurate (0.3 µL, 0.0005 mmol, 0.1 mol-%) in THF (0.5 mL). Afterwards, IPDI (106 µL, 0.5 mmol, 1.0 equiv.) was added in one portion. Stirring was continued at 50 °C for 4 h, followed by addition of H₂O (10 mL) and stirring for 5 min. Afterwards, the reaction solution was extracted with CH₂Cl₂ (3 x 20 mL). The combined organic layers were dried over Na₂SO₄ and concentrated in vacuum to give the titled product as a mixture of isomers and was obtained as a highly-viscous, colorless oil in 82% yield (266 mg, 0.41 mmol).

¹³C{¹H} NMR (75 MHz, DMSO-d₆): δ 157.6, 155.0, 151.5, 87.3, 85.7, 70.6, 46.7, 46.1, 44.1, 43.2, 40.8, 40.5, 36.5, 35.0, 31.8, 29.3, 29.3, 27.6, 26.4, 26.3, 25.6, 23.3, 18.0, 17.6.

Due to the isomeric product mixture, the peak assignment might be inaccurate.

HRMS (APCI): [m/z] calculated for C₃₄H₅₄N₂O₁₀H⁺ ([M+H]⁺): 651.3851; found: 651.3852.

HRMS (APCI): [m/z] calculated for C₃₄H₅₄N₂O₁₀NH₄⁺ ([M+NH₄]⁺): 668.4117; found: 668.4116.

HRMS (APCI): [m/z] calculated for C₃₄H₅₄N₂O₁₀Cl⁻ ([M+Cl]⁻): 685.3472; found: 685.3472.

HRMS (ESI): [m/z] calculated for C₃₄H₅₄N₂O₁₀Na⁺ ([M+Na]⁺): 673.3671; found: 673.3677.

### Conversion of 4-(4-hydroxy-4-methylpentyl)-4-methyl-5-methylene-1,3-dioxolan-2-one with HDI-trimer

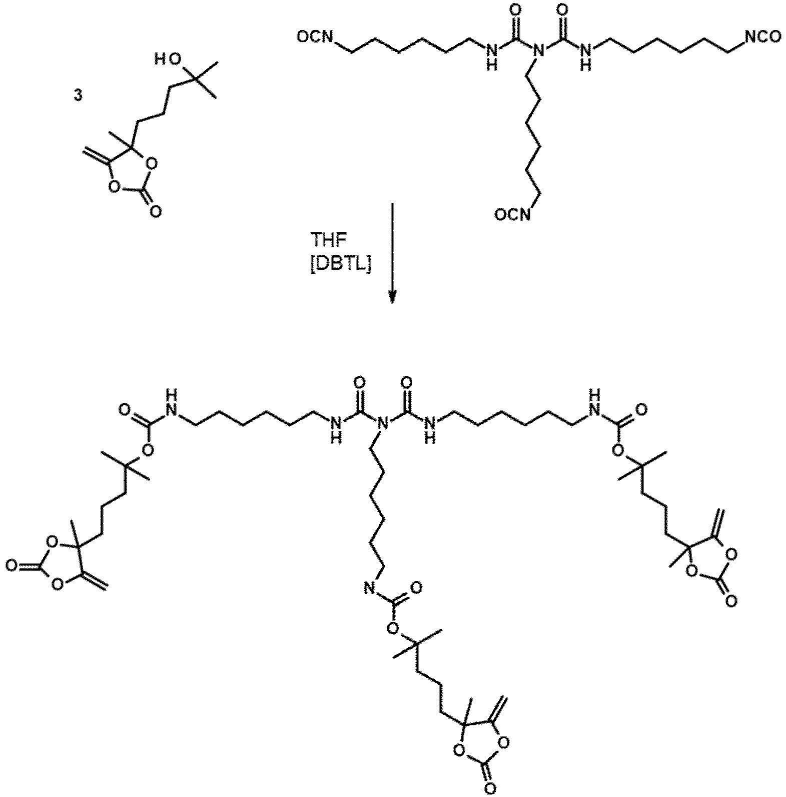

To 80 g of THF, 4-(4-hydroxy-4-methylpentyl)-4-methyl-5-methylene-1,3-dioxolan-2-one (40 g, 187 mmol) and dibutyltin dilaurate (DBTL; 2.36 g, 3,7 mmol) were added, followed by addition of Basonat HI2000 NG (41 g).

The reaction mixture was stirred under Ar at 50 °C for 3 h, followed by stirring over night at room temperature. The reaction mixture was filtered over silica and concentrated in vacuum. A pale yellow resin is obtained which is solid at RT.

¹³C NMR confirmed complete conversion of 4-(4-hydroxy-4-methylpentyl)-4-methyl-5-methylene-1,3-dioxolan-2-one via shift of the signal of the R-**C**(Me)₂-OH [70.8] by the signal for -O-**C**(Me)₂-R [80.4].

¹³C{¹H} NMR (125 MHz, CDCl₃): δ 157.8, 155.7, 151.5, 149.0, 87.1, 85.6, 80.4, 40.3, 29.7, 26.4, 26.3, 26.1, 17.5, 17.1.

### III. Compound(s) of formula (VI)

### Conversion of 4-(4-hydroxy-4-methylpentyl)-4-methyl-5-methylene-1,3-dioxolan-2-one with IPDI and neopentyl glycol

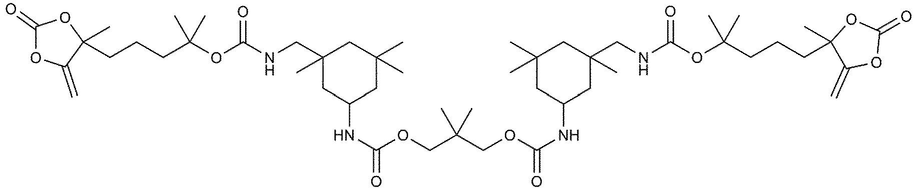

In a 20 mL vial, 4-(4-hydroxy-4-methylpentyl)-4-methyl-5-methylene-1,3-dioxolan-2-one (1.07 g, 5 mmol, 2.0 equiv.) was dissolved in THF (5 mL). Dibutyltin dilaurate (60 µL, 0.1 mmol, 2 mol-%) and isophorone diisocyanate (1.06 mL, 5 mmol, 2.0 equiv.) were added and the reaction mixture was stirred at 50 °C for 2 h. Then, neopentyl glycol (260.6 mg, 2.5 mmol, 1.0 equiv.), dissolved in 1 mL of 1,4-dioxane, was added and stirring was continued at 50 °C for 2 h. Afterwards, H₂O (10 mL) was added and, while stirring, the reaction mixture was allowed to cool down to room temperature. The reaction solution was extracted with CH₂Cl₂ (4 x 10 mL). The combined organic layers were dried over Na₂SO₄ and concentrated in vacuum. The product was received as a white foam that was crushed to give a white powder.

HRMS (ESI): [m/z] calculated for C₅₁H₈₄N₄O₁₄H⁺ ([M+H]⁺): 977.6057; found: 977.6057.

HRMS (ESI): [m/z] calculated for C₅₁H₈₄N₄O₁₄NH₄⁺ ([M+NH₄]⁺): 994.6322; found: 994.6323.

### Conversion of 4-(4-hydroxy-4-methylpentyl)-4-methyl-5-methylene-1,3-dioxolan-2-one with IPDI and trimethylolpropanethoxylat (MW 170)

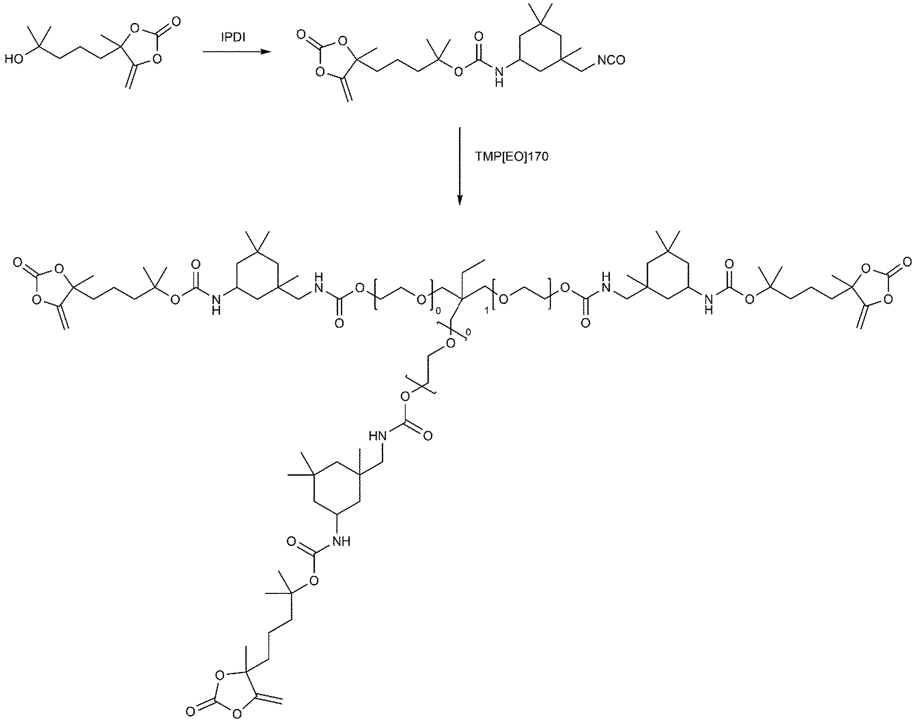

In a 25 mL round-bottomed flask, 4-(4-hydroxy-4-methylpentyl)-4-methyl-5-methylene-1,3-dioxolan-2-one (1.07 g, 5 mmol, 1.0 equiv.) was dissolved in THF (5 mL) and dibutyltin dilaurate (60 µL, 0.1 mmol, 2 mol-%) was added, followed by addition of isophorone diisocyanate (1.06 mL, 5 mmol, 1.0 equiv.). The reaction mixture was stirred at 50 °C for 2 h. Then, TMP-ethoxylate (Mₙ ~ 170 g/mol, 283 mg, 1.66 mmol, 0.33 equiv.) dissolved in ~5 mL THF was added. The reaction mixture was stirred at 50 °C for 2 h. Afterwards, activated charcoal (tip of spatula) was added and the reaction mixture was filtered through a plug of SiO₂. After concentration, the product was received as a white foam that was crushed to give a white powder. NMR analysis showed traces of remaining ISO-groups.

### Conversion of 4-(4-hydroxy-4-methylpentyl)-4-methyl-5-methylene-1,3-dioxolan-2-one with IPDI and trimethylolpropanethoxylat (MW 1014)

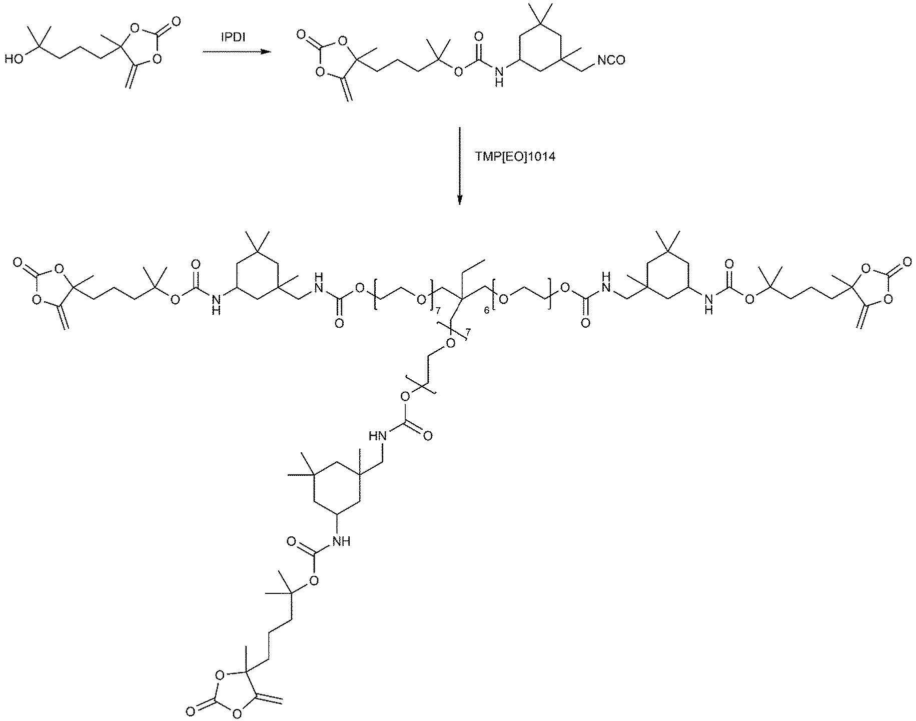

In a 250 mL round-bottomed flask, 4-(4-hydroxy-4-methylpentyl)-4-methyl-5-methylene-1,3-dioxolan-2-one (10.7 g, 50 mmol, 1.0 equiv.) was dissolved in THF (50 mL) and dibutyltin dilaurate (600 µL, 1 mmol, 2 mol-%) was added, followed by addition of isophorone diisocyanate (11.1 g, 50 mmol, 1.0 equiv.). The reaction mixture was stirred at 50 °C for 3 h. Then, TMP-ethoxylate (Mₙ ~ 1014 g/mol, 17.1 g, 16.8 mmol, 0.34 equiv.) dissolved in ~150 mL THF was added. The reaction mixture was stirred at 50 °C over the weekend (3 days) and afterwards filtered through a plug of SiO₂ using MeCN as solvent. After concentration, the product was received as a highly-viscous, slightly yellowish oil. NMR analysis showed no more presence of remaining ISO-groups.

Titration with N,N-dibutylamine confirmed the presence of 2.6 functional groups per molecule, which is in good agreement with the expected product.

### IV. Model reactions

Bis(2-methyl-5-(4-methyl-5-methylene-2-oxo-1,3-dioxolan-4-yl)pentan-2-yl)hexane-1,6-diyldicarbamate was reacted with a primary amine (n-butylamine) and a secondary amine (pyrrolidine) in model reactions.

### Reaction with n-butvlamine

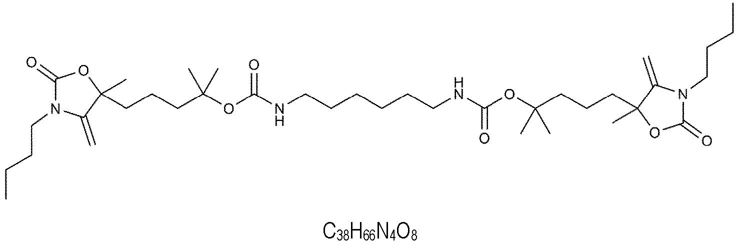

To bis(2-methyl-5-(4-methyl-5-methylene-2-oxo-1,3-dioxolan-4-yl)pentan-2-yl)hexane-1,6-diyldicarbamate, 1 mL of n-butylamine was added. The reaction mixture was stirred at room temperature for 4 h. Then, CH₂Cl₂ (20 mL) was added, followed by extracting with 1M HCl (3 x 30 mL). The organic layer was dried over Na₂SO₄ and concentrated in vacuum to give the product as a highly viscous, colorless oil.

¹³C{¹H} NMR (75 MHz, DMSO-d6): δ 157.0, 155.4, 151.0, 87.5, 86.0, 79.0, 30.1, 29.5, 26.4, 26.1, 26.1, 26.0, 25.5, 17.2.

HRMS (ESI): [m/z] calculated for C₃₈H₆₆N₄O₈Na⁺ ([M+Na]⁺): 729.4773; found: 729.4785.

### Reaction with pyrrolidine

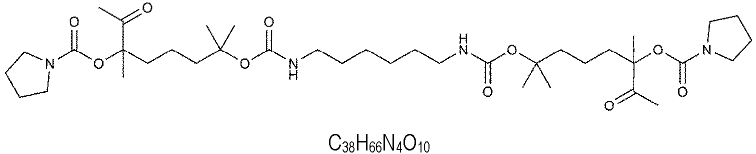

To bis(2-methyl-5-(4-methyl-5-methylene-2-oxo-1,3-dioxolan-4-yl)pentan-2-yl)hexane-1,6-diyldicarbamate, 1 mL of pyrrolidine was added. The reaction mixture was stirred at room temperature for 4 h. A yellow reaction mixture was obtained. After 4 h, CH₂Cl₂ (20 mL) was added, followed by extracting with 1M HCl (3 x 30 mL). The organic layer was dried over Na₂SO₄ and concentrated in vacuum to give the product as a highly viscous, yellowish oil.

¹³C{¹H} NMR (75 MHz, DMSO-d6): δ 157.0, 155.4, 151.0, 87.5, 86.0, 79.0, 30.1, 29.5, 26.4, 26.1, 26.1, 26.0, 25.5, 17.2.

HRMS (ESI): [m/z] calculated for C₃₈H₆₆N₄O₁₀Na⁺ ([M+Na]⁺): 761.4671; found: 761.4679.

HRMS (ESI): [m/z] calculated for C₃₈H₆₆N₄O₁₀Cl⁻ ([M+Cl]⁻): 773.4473; found: 773.4492.

## Claims

1. A compound of formula (I) 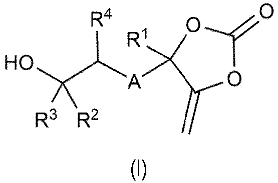 wherein
R¹ is H or methyl,
R² and R³ are independently selected from H, C₁-C₄ alkyl, cycloalkyl and phenyl-C₁-C₄-alkyl, or
R² and R³, together with the carbon atom to which they are attached, form a C₃-C₆ carbocycle,
R⁴ is H or C₁-C₄ alkyl, and
A is C₂-C₇-alkylene.

2. The compound of claim 1, which is

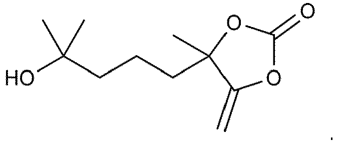

3. A method for preparing a compound of formula (I) of claim 1, comprising hydrating a compound of formula (II) 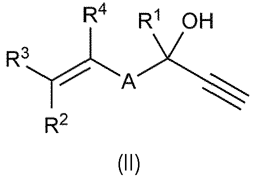 to obtain a compound of formula (III) 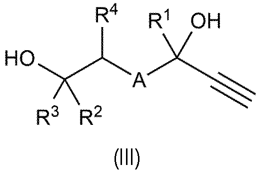 and reacting the compound of formula (III) with carbon dioxide to obtain the compound of formula (I).

4. A compound of formula (IV) 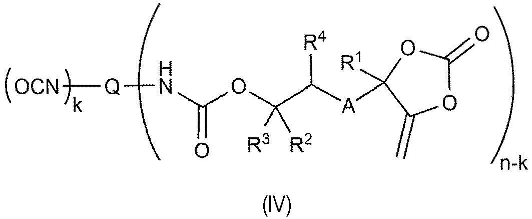 wherein
Q is the residue of an n functional polyisocyanate,
n is from 1.5 to 3,
k is from 0 to n-1,
R¹ is H or methyl,
R² and R³ are independently selected from H, C₁-C₄ alkyl, cycloalkyl and phenyl-C₁-C₄-alkyl, or
R² and R³, together with the carbon atom to which they are attached, form a C₃-C₆ carbocycle,
R⁴ is H or C₁-C₄ alkyl, and
A is C₂-C₇-alkylene.

5. The compound of claim 4, wherein R¹, R² and R³ are methyl, R⁴ is H, and A is -(CH₂)₂.

6. The compound of claim 4 or 5, wherein Q is the residue of an n functional monomeric polyisocyanate or an n functional oligomeric polyisocyanate.

7. The compound of claim 6, wherein Q is the residue of
an n functional monomeric polyisocyanate which is selected from
- pentamethylenediisocyanate (PDI),
- hexamethylenedisocyanate (HDI),
- dicyclohexylmethane-4,4'-diisocyanate (H12MDI),
- isophorone diisocyanate (IPDI),
- dimeryl diisocyanate (DDI),
- toluene diisocyanate (TDI),
- 4,4'-methylene diphenyl diisocyanate (MDI),
- naphthalene diisocyanate (NDI),
- m-xylylenediisocyanate (XDI),
or
an n functional oligomeric polyisocyanate which is selected from
- oligomeric MDI (also referred to as "polymeric MDI" (PMDI)),
- polyisocyanates having uretdione groups,
- polyisocyanates having isocyanurate groups,
- polyisocyanates having biuret groups,
- polyisocyanates having urethane groups or allophanate groups,
- polyisocyanates comprising oxadiazinetrione groups,
- uretonimine-modified polyisocyanates,
- carbodiimide-modified polyisocyanates,
- polyurethane-polyisocyanate prepolymers or polyurea-polyisocyanate prepolymers,
or combinations of these polyisocyanates.

8. A method for preparing a compound of formula (IV) as defined in any one of claims 4 to 7, comprising reacting an n functional polyisocyanate of formula (V) 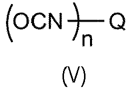 wherein
Q and n are as defined in claims 4 to 7,
with a compound of formula (I) as defined in claim 1 or 2 to obtain the compound of formula (IV).

9. A compound of formula (VI) 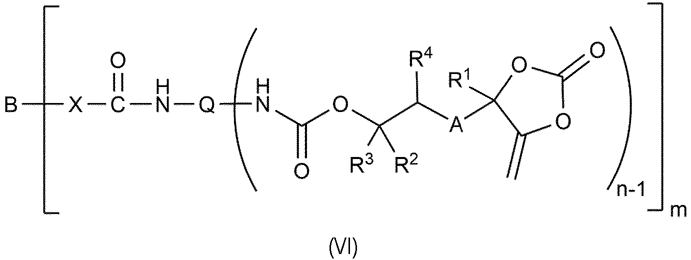 wherein
B is an m functional residue of a compound having m isocyanate-reactive groups,
X is O, S or NE, wherein E is H or C₁-C₄ alkyl,
m is from 2 to 8,
Q is the residue of an n functional polyisocyanate,
n is from 1.5 to 3,
R¹ is H or methyl,
R² and R³ are independently selected from H, C₁-C₄ alkyl, cycloalkyl and phenyl-C₁-C₄-alkyl, or
R² and R³, together with the carbon atom to which they are attached, form a C₃-C₆ carbocycle,
R⁴ is H or C₁-C₄ alkyl, and
A is C₂-C₇-alkylene.

10. The compound of claim 9, wherein B is an m functional residue of a compound having m isocyanate-reactive groups selected from polyols, polyamines, alkanolamines, and polythiols, and mixtures thereof.

11. A method for preparing a compound of formula (VI) as defined in claim 9 or 10, comprising reacting a compound having m isocyanate-reactive groups of formula (VII) 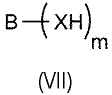 wherein m, B and X are as defined in claim 9,
with a compound of formula (IV) as defined in claim 4 with k = 1 to obtain the compound of formula (VI).

12. A radically polymerizable compound of the formula (VIII) 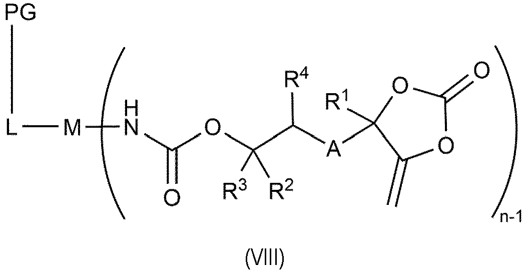 wherein
M is 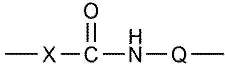 or a chemical bond,
PG is a radically polymerizable group,
L is a linker,
X is O, S or NE, wherein E is H or C₁-C₄ alkyl,
Q is the residue of an n functional polyisocyanate,
n is from 1.5 to 3,
R¹ is H or methyl,
R² and R³ are independently selected from H, C₁-C₄ alkyl, cycloalkyl and phenyl-C₁-C₄-alkyl, or
R² and R³, together with the carbon atom to which they are attached, form a C₃-C₆ carbocycle,
R⁴ is H or C₁-C₄ alkyl, and
A is C₂-C₇-alkylene.

13. The compound of claim 12, wherein PG is a (meth)acryloyloxy group, L is C₂-C₆ alkylene, and X is O.

14. A polymeric compound, comprising a polymeric backbone comprised of a plurality of backbone units, of the formula (IX): 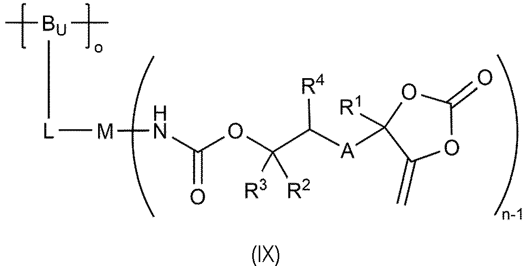 wherein
M is 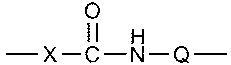
or a chemical bond,
Bu is a backbone unit within the polymeric backbone,
o is 2 to 50,
L is a linker,
X is O, S or NE, wherein E is H or C₁-C₄ alkyl,
Q is the residue of an n functional polyisocyanate,
n is from 1.5 to 3,
R¹ is H or methyl,
R² and R³ are independently selected from H, C₁-C₄ alkyl, cycloalkyl and phenyl-C₁-C₄-alkyl, or
R² and R³, together with the carbon atom to which they are attached, form a C₃-C₆ carbocycle,
R⁴ is H or C₁-C₄ alkyl, and
A is C₂-C₇-alkylene.

15. A composition comprising a compound of any one of claims 1, 2, 4 to 7, 9, 10, or 12 to 14.
